Europäisches Patentamt

European Patent Office

Office européen des brevets

(11) Numéro de publication: **0 262 029**
**A1**

## DEMANDE DE BREVET EUROPEEN

(21) Numéro de dépôt: **87402047.2**

(51) Int. Cl.⁴: **A 61 M 5/00**

(22) Date de dépôt: **15.09.87**

(30) Priorité: **16.09.86 FR 8612912**

(43) Date de publication de la demande:
**30.03.88 Bulletin 88/13**

(84) Etats contractants désignés: **BE DE GB IT NL**

(71) Demandeur: **Dang, Thi**
**27, rue Hamelin**
**F-75116 Paris (FR)**

**Supernant, Ginette**
**6, Square Léon Guillot**
**F-75015 Paris (FR)**

**Duval, Paulette**
**6, Cours Marigny**
**F-94300 Vincennes (FR)**

(72) Inventeur: **Dang, Thi**
**27, rue Hamelin**
**F-75116 Paris (FR)**

**Supernant, Ginette**
**6, Square Léon Guillot**
**F-75015 Paris (FR)**

**Duval, Paulette**
**6, Cours Marigny**
**F-94300 Vincennes (FR)**

(54) Dispositif de fixation et de positionnement d'une seringue, et application à la réalisation de cholangiographie.

(57) L'invention a trait à un dispositif de fixation et de positionnement d'une seringue, du type de ceux comprenant des anneaux ouverts (21, 21', 21″) disposés sur une tige centrale (20) et dans lesquels vient s'insérer ladite seringue (30).

Le dispositif, selon la présente invention, est remarquable en ce qu'il comprend en outre une patte de fixation (22) composée de plusieurs doigts (23, 23', 23″) disposés à une première extrémité de cette patte, alors que l'autre extrémité est reliée d'une manière fixe à ladite tige centrale.

FIG.2a

EP 0 262 029 A1

## Description

Dispositif de fixation et de positionnement d'une seringue, et application à la réalisation de cholangiographie.

La présente invention a trait à un dispositif de fixation et de positionnement d'une seringue, du type de ceux comprenant des anneaux ouverts disposés sur une tige centrale et dans lesquels vient s'insérer ladite seringue.

L'invention concerne la médecine humaine, et plus spécifiquement la radiographie médicale, notamment l'exploration per opératoire radiographique des voies biliaires (ou cholangiographie).

La cholangiographie est un examen de routine en cas d'ablation de la vésicule (ou cholecystectomie). Elle est essentielle car elle permet de révéler l'existence de calculs dans le cholédoque, qui peuvent être fatals pour le malade.

L'exploration manuelle de la voie biliaire principale a été longtemps pratiquée. Elle consiste à explorer celle-ci entre le pouce antérieur et les autres doigts insinués dans l'orifice de l'hiatus de Winslow, et ainsi de déceler la saillie d'un ou de plusieurs calculs. L'inconvénient majeur de l'exploration manuelle est de laisser échapper les petits calculs, ainsi que d'en chasser certains mobiles vers les voies intra-hépatiques.

Par la suite, est apparue une méthode radiomanométrique d'exploration de la voie biliaire, que l'on attribue à CAROLI. Elle est décrite notamment dans le Nouveau Traité de Technique Chirurgicale, Tome II, Fascicule 2 - Voies biliaires - p. 82 et suivantes, de Patel et alii, chez MASSON.

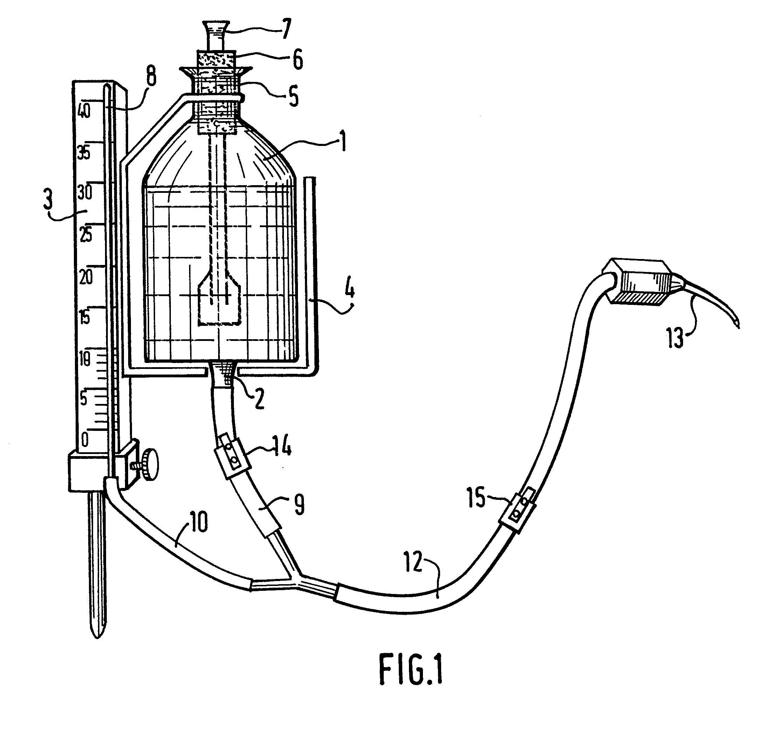

Cet appareil, tel que décrit en référence à la Figure 1, se compose d'un flacon (1) muni en son fond d'un embout (2) et qui est disposé le long d'une règle graduée (3), dans une nacelle (4) montée coulissante. Dans le col (5) du flacon (1), un bouchon percé (6) laisse passer une pipette (7) à cols évasés, qui communique à l'air libre. Un tube (8), dont l'extrémité supérieure est libre, est disposé de manière fixe le long de la règle graduée (3). L'embout (2) du flacon et le tube (8) sont reliés par des tubes caoutchoutés (resp. 9 et 10) à un tube en Y (11), lui-même relié par un tube caoutchouté (12) à une canule (13). Des moyens de fermeture (tels que des clamps) permettent d'isoler le flacon perfuseur (14) ou tout le système (15).

La radiomanométrie des voies biliaires consiste à injecter dans celles-ci, une substance de contraste, typiquement une solution aqueuse iodée type Diodone, sous une pression facilement mesurable, et dont le niveau est automatiquement compensé et entretenu, quel que soit le débit d'injection.

L'appareil est monté de favon fixe, à côté de l'opérateur, de telle sorte que le niveau 0 de la règle graduée corresponde approximativement au niveau du pédicule hépatique. Puis l'appareil est rempli de la solution de contraste désirée, le flacon étant disposé en haut de la règle graduée de telle sorte que tout l'air soit chassé de l'appareillage. Le flacon est ensuite descendu jusqu'à ce que le niveau du liquide dans le tube (8) soit au zéro : le tube (8) fonctionne alors comme un manomètre. Le tuyau caoutchouté (12) peut alors être ouvert.

Par évaluation du flacon perfuseur (1) le long de la règle graduée (3) qui communique avec le tube (8), on augmente la pression du liquide de contraste, et on mesure cette pression par simple lecture du niveau du liquide dans le tube (8), en regard de la règle graduée (3).

Conformément aux explications données dans l'ouvrage précité, auquel un lecteur attentif voudra bien se rapporter pour plus de détails, on peut ainsi mesurer la pression de remplissage, lorsque le liquide de contraste commence à pénétrer dans le cholédoque, la pression de passage, lorsque le liquide de contraste commence à franchir le sphincter d'Oddi, et la pression résiduelle, lorsqu'on interrompt la perfusion.

Ces mesures de pression sont doublées par des clichés radiographiques, qui permettent d'apprécier alors l'intégrité des voies biliaires et de déceler la présence de calculs.

Cet appareil est de nos jours tombé en désuétude, car son maniement (mise en place et manipulations opératoires) est difficile. Cet appareil est en verre, donc fragile et coûteux, et il est nécessaire de le stériliser à chaque usage. En outre, l'intérêt de la manométrie est très discuté, car le rôle dévolu au sphincter d'Oddi a été récemment controversé.

Il n'y a donc plus aujourd'hui de système à usage simple, permettant de faire une radiographie pendant une cholecystectomie, et les chirurgiens ont alors recours à des appareils ou manoeuvres personnelles, certainement efficaces mais qui n'ont pas la rigueur d'un système standardisé.

L'invention vise à proposer un appareil simple et d'usage unique, permettant de pallier les susdits inconvénients.

Le dispositif selon la présente invention est remarquable en ce qu'il comprend en outre une patte de fixation composée de plusieurs doigts disposès à une première extrémité de cette patte, alors que l'autre extrémité est reliée d'une manière fixe à ladite tige centrale.

De cette manière, la patte de fixation peut être solidarisée à un support, généralement plan, au niveau de ses doigts, par tout moyen convenable, tel qu'une bande adhésive ou autre, et fixer ainsi, d'une manière simple et efficace, l'ensemble constitué par la tige centrale munie de ses anneaux, et qui joue le rôle de porte-seringue.

Selon une première variante de l'invention, le dispositif de fixation est également un dispositif de positionnement vertical d'une seringue, lorsque la patte de fixation et les doigts sont contenus dans un plan perpendiculaire à la tige centrale et qu'elle comprend en outre une butée d'arrêt disposée sur la patte de fixation, de manière à positionner la tige centrale à une distance prédéterminée d'un objet, présentant un plan de fixation horizontal sur lequel on dispose les doigts et on les y rend solidaires au moyen d'un élément adhésif.

De cette manière, on peut alors disposer le porte-seringue, et donc la seringue, d'une façon verticale, ce qui facilite sa préhension et l'injection du liquide y contenu.

Selon une réalisation de l'invention, un appareil d'injection d'un liquide de contraste destiné à permettre des radiographies médicales peut être réalisé à partir d'une seringue d'injection dans laquelle est disposé le liquide de contraste, d'une tubulure souple et d'une canule d'injection, ladite seringue étant disposée dans un appareil de fixation et/ou de positionnement, comme décrit ci-dessus.

De cette manière, le praticien dispose d'un appareil lui permet tant de réaliser des radiographies per opératoires, d'un usage simple, et qui peut en outre être rendu stérile et donc à usage unique.

Les figures données en annexe permettront de mieux comprendre l'invention, et de mieux en apprécier sa portée.

La figure 1 représente l'appareil radiomanométrique de Caroli, et a été décrite précédemment.

La figure 2 représente le dispositif de fixation et/ou de positionnement vertical, vu de côté (2a) et de dessus (2b).

La figure 3 représente l'appareil d'injection, dans son ensemble.

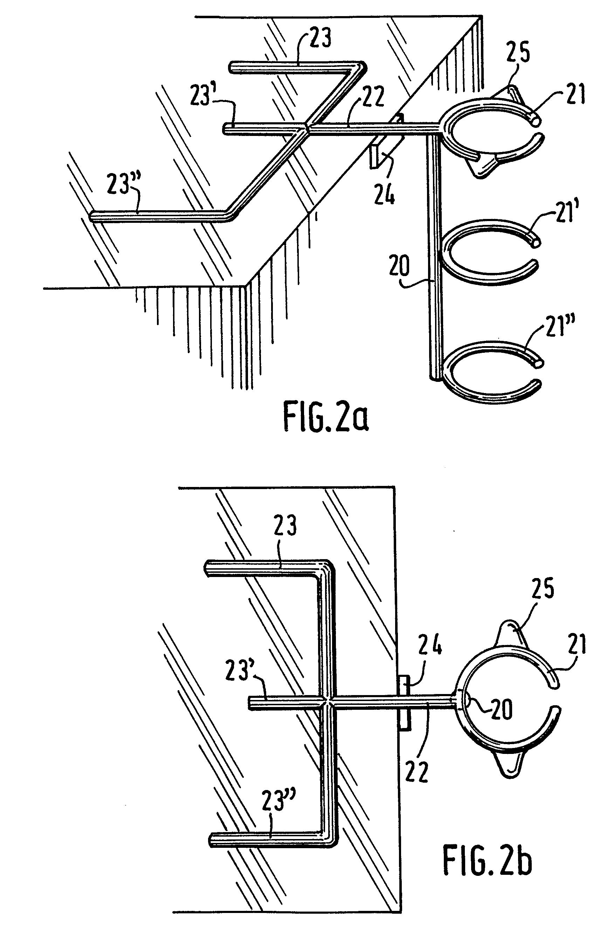

En référence à la figure 2, une tige centrale (20) sur laquelle sont disposés des anneaux ouverts (21, 21', 21") est reliée à une extrémité d'une patte de fixation (22), alors qu'à son autre extrémité, sont disposés plusieurs doigts (23, 23', 23").

Selon une variante, une butée d'arrêt (24) permet de positionner l'ensemble à une distance prédéterminée d'un support.

Si la patte de fixation et les doigts sont contenus dans un plan perpendiculaire à la tige centrale, ainsi que représenté ici et notamment sur la vue de dessus (2b), on a alors un moyen simple pour positionner de façon verticale une seringue.

Dans ces deux figures (2a et 2b), les éléments identiques portent les mêmes références. Ces divers éléments peuvent être réalisés en tout matériau adéquat, par exemple en matière plastique, ou en plastique armé...

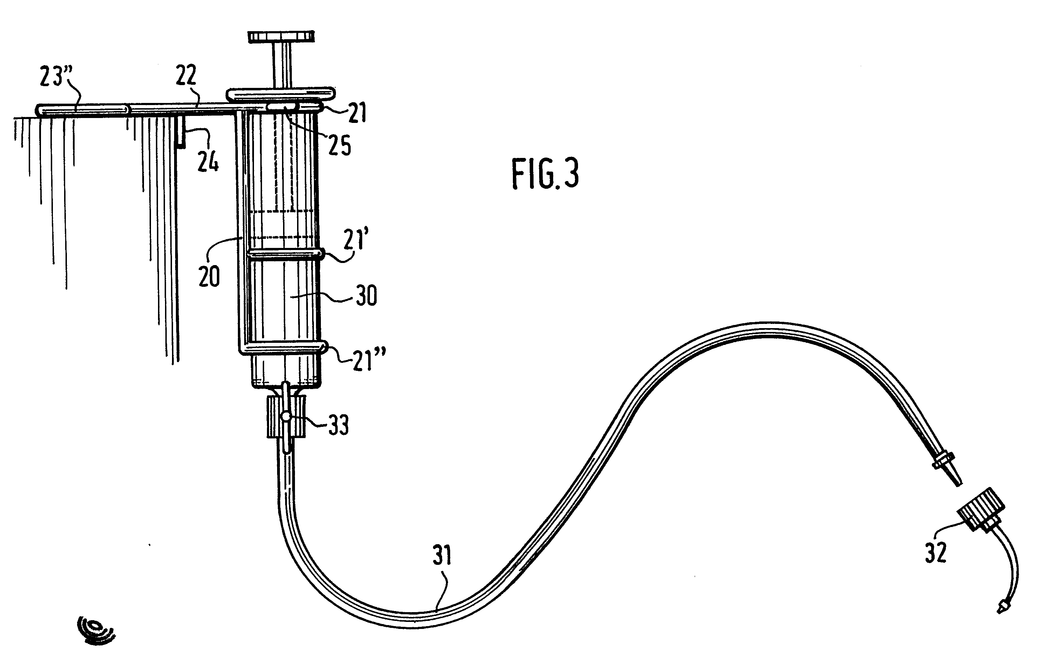

Enfin, la figure 3 représente un appareil d'injection d'un liquide de contraste, destiné par exemple à réaliser des radiographies médicales per opératoires, telles que des cholangiographies ou autres ; il se compose du dispositif tel que décrit à la figure 2 précédente, dans lequel on vient insérer une seringue d'injection (30), remplie d'un liquide de contraste adéquat, et muni en son bout d'une tubulure souple (31), terminée par une canule d'injection (32), de type standard. Un robinet (33), placé entre la seringue et la tubulure, permet d'isoler celle-ci et de contrôler le flux du liquide de contraste.

Dans une application particulière de cet appareil, par exemple pour la réalisation d'une cholangiographie, il suffira au chirurgien de mettre en place la canule dans le canal cystique. Le support est fixé sur une table, par la bande adhésive, et la seringue est placée dans le support et reliée à la tubulure. Le robinet étant fermé, la seringue est remplie du liquide de contraste ; puis, le système seringue-tubulure est purgé. Après raccordement de la tubulure à la canule transcystique, on met en place le piston de la seringue. Après vérification du bon fonctionnement et positionnement de l'amplificateur de brillance, on ouvre le robinet et on injecte le liquide de contraste par action sur le piston de la seringue, de manière à réaliser les clichés radiographiques en cause.

Afin de faciliter l'injection du liquide de contraste, l'anneau supérieur (21) disposé sur la tige centrale (20) pourra être utilement muni d'oreilles (25) destinées à faciliter sa préhension et le blocage de la seringue dans cet ensemble porte-seringue, par les doigts du manipulateur.

Cet ensemble peut être livré au praticien de façon stérile, en le disposant dans des emballages semi-hermétiques et en le soumettant à un traitement de stérilisation connu en soi. Il est d'un maniement simple, et se fixe sur tout support-plan horizontal que l'on trouve dans les enceintes opératoires, et de par la relative modicité de son coût de revient, il peut se jeter après chaque usage, ce qui limite d'autant la diffusion de germes pathogènes indésirables que l'on trouve parfois dans les hôpitaux.

Les modes de réalisation qui viennent d'être décrits ne sont donnés qu'à titre indicatif, et d'autres mises en oeuvre de l'invention, à la portée de l'Homme de l'Art, pourraient être adoptées sans pour cela faire oeuvre inventive, et donc sans sortir du cadre de la présente invention.

**Revendications**

1.- Dispositif de fixation d'une seringue, du type comprenant des anneaux ouverts (21) disposés le long d'une tige centrale (20), dans lesquels vient s'insérer ladite seringue, caractérisé en ce qu'il comprend en outre une patte de fixation (22) composée de plusieurs doigts (23) disposés à une première extrémité de cette patte, alors que l'autre extrémité est reliée d'une manière fixe à ladite tige centrale.

2.- Dispositif de fixation et de positionnement vertical d'une seringue, selon la revendication 1, caractérisé en ce que la patte de fixation et les doigts sont contenus dans un plan perpendiculaire à ladite tige centrale, et qu'il comprend en outre une butée d'arrêt (24), disposée sur la patte de fixation, de manière à positionner la tige centrale à une distance prédéterminée d'un objet, présentant un plan de fixation horizontal sur lequel on dispose les doigts et on les y rend solidaires au moyen d'un élément adhésif.

3.- Appareil d'injection d'un liquide de contraste, destiné à permettre des radiographies médicales, constitué d'une seringue d'injection (30) dans laquelle est disposé le liquide de contraste, d'une tubulure souple (31) et d'une canule d'injection (32), caractérisé en ce que ladite seringue est disposée dans un appareil de fixation selon l'une des revendications 1 et 2.

4.- Appareil d'injection d'un liquide de contraste, selon la revendication 3, caractérisé en ce qu'il est disposé dans une enveloppe semi-hermétique, soumis à un traitement de stérilisation et commercialisé tel quel.

FIG.1

0262029

FIG.2a

FIG.2b

FIG.3

# RAPPORT DE RECHERCHE EUROPEENNE

Office européen
des brevets

Numero de la demande

EP 87 40 2047

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-3 529 598 (WALDHAM) <br> * Résumé; figure 2 * <br> --- | 1,2 | A 61 M 5/00 |
| A | EP-A-0 176 241 (J.B. HARPER) <br> * Résumé; figures * <br> --- | 1 | |
| A | US-A-4 044 757 (McWHORTER) <br> * Description; figures * <br> ----- | 1,3 | |

DOMAINES TECHNIQUES
RECHERCHES (Int. Cl.4)

A 61 M

Le présent rapport a été établi pour toutes les revendications

| Lieu de la recherche | Date d'achèvement de la recherche | Examinateur |
|---|---|---|
| LA HAYE | 18-12-1987 | VANRUNXT J.M.A. |

EPO FORM 1503 03.82 (P0402)